# EUROPEAN PATENT APPLICATION

(11) **EP 4 635 564 A1**
(43) Date of publication of application: **22.10.2025**
(21) Application number: 24382399.4
(22) Date of filing: 16.04.2024
(51) Int. Cl.: A61N 7/00, A61B 90/50

(54) **ADJUSTABLE STRETCHER AND SYSTEM FOR SPINAL TREATMENT AND METHOD FOR OPTIMALLY CONFIGURING SAID SYSTEM**

(71) Applicant: Universitat Pompeu Fabra, 08002 Barcelona (ES); Fundació Institut Hospital del Mar d'Investigacions Mèdiques, 08003 Barcelona (ES)
(72) Inventor: GARCIA OJALVO, Jorge, 08002 Barcelona (ES); CONESA CELDRAN, Agustín Fernando, 08002 Barcelona (ES); VILLOSLADA DIAZ, Pablo, 08003 Barcelona (ES)
(74) Representative: TRBL Intellectual Property

(57) **Abstract**

The present invention discloses an adjustable stretcher (1) adapted to fit a subject lying in a supine position. The stretcher comprises a plurality of supporting modules (2), whose relative positions can be adjusted according to the dimensions of said subject. Furthermore, the stretcher (1) comprises an opening (8) in the craniocaudal direction of the subject so that the spinal region of the subject may be fitted within the opening (8), and which is adapted with a holder (9), in which a spinal region treatment device (10) can be mounted. A system (16) for non-invasive neurostimulation of the spinal cord comprising said stretcher (1) and wherein the spinal region treatment device (10) corresponds to one or more Focused Ultrasound (FUS) transducer arrays (17) composed by circular arrays of piezoelectric elements and adapted to be applied on the skin of a subject and arranged laterally to the posterior midline of the subject's back so that the FUS beam is steered through the vertebrae's lamina (19) is also disclosed. The system (16) further comprises a control unit (18) providing the power and software means for positioning the subject and for managing the stimulation parameters. Finally, a method for optimizing the configuration of said system (16) is disclosed.

## Description

### FIELD OF THE INVENTION

The present invention belongs to the field of medical devices and apparatuses, and, more specifically, to a stretcher adapted to hold a subject lying in a supine position with its spinal region securely placed in an opening of said stretcher arranged at the craniocaudal direction. The opening of the stretcher comprises a holder adapted to receive a spinal region treatment device, and, more specifically, a focused ultrasound (FUS) transducer array aimed at stimulation of the spinal cord. A system comprising both the stretcher, the FUS transducer arrays and a control unit for optimizing the configuration of said system is disclosed, as well as a method for optimal system configuration.

### BACKGROUND OF THE INVENTION

Neurostimulation is the purposeful modulation of the nervous system's activity using invasive or non-invasive means, usually with the aim of treating brain or central nervous system (CNS) disorders. Within this framework, several techniques have been tested, such as electric (e.g., transcranial direct current stimulation (tDCS)), magnetic (transcranial magnetic stimulation (TMS)), or light (near-infrared stimulation (NIR)) stimulation. However, such approaches have failed to deliver significant benefits due to their low penetration, only reaching superficial cortical neurons, and due to their poor spatial resolution. For these reasons, none of these technologies, except spinal electric stimulation, is suitable to stimulate the spinal cord.

Focused Ultrasound (FUS) is a non-invasive therapeutic technique that consists of delivering the mechanical energy of sound waves to a target tissue or body region with high spatial resolution. Its effects on different tissues may be related to several physical processes such as heating, acoustic cavitation, radiation force (also known as acoustic streaming), or the effects of microbubbles.

Relating to treatments of the Central Nervous System (CNS), the use of FUS techniques allows for targeting regions with a diameter of 1-2 mm. Furthermore, this approach has deep penetration capabilities, so its use also enables for reaching deep brain structures (such as the brainstem), and the spinal cord. Beyond these characteristics, since ultrasounds have been used for diagnosis for decades, their associated safety parameters are well known in clinical circumstances.

FUS can be divided into two categories, depending on the intensity of the delivered sound waves: high-intensity FUS (HIFUS) and low-intensity FUS (LIFUS). On the one hand, HIFUS, i.e., FUS with intensities larger than 200 W/cm2, is often used for heating the target tissue, and has been used for lithotripsy, tissue ablation, cancer therapy or idiopathic tremor, as well as for treating Parkinson's disease. LIFUS, i.e., FUS with intensities lower than 10 W/cm2, on the other hand, can modulate cell activity primarily by radiation force effects, which represent the nanoscale movement of cell membranes due to the sound waves, inducing mechanoreceptor activation (e.g., transient receptor potential (TRP) and Ca channels such as Piezo 1 and 2) and membrane protein complex activation.

LIFUS can activate several mechanoreceptors both in the CNS, and in the peripheral nervous system (PNS), such as Piezo1, Piezo2 and TRPs. Piezo1 is present in astrocytes mediating calcium oscillations and cytokines release in oligodendrocyte precursor cells, which sense the stiffness of the tissue and define the ability to regenerate the CNS. It is also present in axons, promoting regeneration, and in the immune system, such as in macrophages, activating the innate immune response to pressure changes due to diseases, suggesting that microglia may also be responsive to LIFUS. Studies in several animal models of CNS damage support the use of LIFUS for preventing brain damage and for providing protection against neuroinflammation and memory impairments in models of Alzheimer's disease.

Regarding the role of LIFUS in remyelination, several studies have shown a role for promoting remyelination in peripheral nerve damage. As a treatment for CNS demyelination, such as the one that takes place in patients with Multiple Sclerosis (MS), FUS stimulation of the corpus callosum in the cuprizone model can be used to promote remyelination, using a stimulation protocol that mimics the temporal pattern that promotes neural activation and oligodendrogenesis, achieved by optogenetics manipulation. In addition, LIFUS is able to induce the homeostatic microglia phenotype, which enhances myelin or aggregated protein phagocytosis and reduces pro-inflammatory microglia and the formation of non-supportive glial scars. Thus, this evidence provides a framework for the role of LIFUS influence in the activation of the primary cell populations involved in brain damage and recovery in MS.

Due to the above-mentioned advantages, FUS, and LIFUS in particular, have been used for treating several conditions of the CNS. Nevertheless, the known implementations of FUS techniques in said treatments relate mainly to brain damage, i.e., to transcranial delivery of FUS. Regarding the use of (LI)FUS for the specific anatomy of the spinal cord, recent studies have provided new stimulation protocols that account for human vertebrates' anatomy, allowing stimulating the spinal cord. However, none of such approaches has delivered useful stimulation prototypes to stimulate the spinal cord and achieve repair and clinical improvement.

Furthermore, many of the proposed systems for spinal cord treatment are focused on blood-spinal cord barrier opening (BSCBO), aimed at facilitating drug entry into the spinal cord or the release of toxic proteins, whereas stimulation, i.e., not BSCBO, has been typically disregarded.

On the other hand, one of the key aspects of the delivery of FUS waves to stimulate the spinal cord relates to the positioning of the ultrasound transducers with respect to the subject. The high spatial resolution of this kind of treatments demands a precise arrangement of the FUS beam/s so that the desired point or region is stimulated. The positioning of the ultrasound transducers has been typically carried out by introducing the subject and the ultrasound delivering system into an MRI or similar machine, so that the alignment of the ultrasound beam and the anatomical region of interest can be performed online. However, this requires a system adapted to be fitted into said MRI or similar machine, and, consequently, the acceptance of the therapy device, cost, and complexity of the procedures increase. Moreover, this is not only true for the delivery of FUS-based treatments, but also for other focal treatments such as radiofrequency or electrical stimulation.

Nevertheless, the offline case also presents some limitations, specially when identifying relevant anatomical landmarks within the patient's body. Palpation techniques have long been used to localize spinal vertebrae levels, especially when doing procedures such as Lumbar Puncture, but different studies have reported mixed results on their accuracy. A common technique used to localize L4 or L4-L5 interspace is the Intercristal (Tuffier) line. Despite its wide use, this line is highly variable, ranging from L1-L2 to L5-S1 (see [Drew Brotherston et al; "Vertebral level identified by the intercristal line: Confirmation by ultrasound"; European Journal of Internal Medicine 86 (Apr. 2021), pp. 118-120; doi: 10.1016/j.ejim.2021.01.015]) leading to Intercristal line of the L4 or L4-L5 level to actually correspond to L3 or L3-L4 levels in 77.3% of cases (see [Robin Chakraverty et al; "Which spinal levels are identified by palpation of the iliac crests and the posterior superior iliac spines?"; Journal of Anatomy 210.2 (Feb. 2007), pp. 232-236; doi: 10.1111/j.1469-7580.2006.00686.x]).

Another reference that is used as anatomical landmark is the posterior Superior Iliac Spine line, which is commonly thought to localize the S2 level. Nevertheless, this line may be identified as S2 in only 51% of the cases and as S1 in 44% of them. A more accurate landmark seems to be the C7, although even this one has an accuracy of only 76% with a correct technique. In general, even anesthetists with over 5 years of experience appear to have trouble with identifying correct lumbar levels, with reporting an accuracy of 29%. Factors that influence these landmarks have been related to gender, abdominal circumference, BMI and age and/or the presence of spinal pathologies.

It is therefore advantageous to provide, first, an apparatus that allows for an optimal positioning of a spinal region treatment device (especially if said device is FUS-based) with respect to the subject to be treated so that anatomical landmarks can be correctly established and maintained, and second, a system for non-invasive and efficient neurostimulation of the spinal cord.

### SUMMARY OF THE INVENTION

The present invention aims to overcome the above-mentioned limitations of the state-of-the-art by providing, first, an adjustable stretcher adapted to fit comfortably a subject lying in a supine position. The stretcher comprises a plurality of supporting modules, whose relative positions can be adjusted according to the dimensions of said subject. Furthermore, the stretcher comprises an opening in the craniocaudal direction of the subject so that the spinal region of the subject may be fitted within the opening. Advantageously, the opening is adapted with a holder in which a spinal region treatment device can be mounted.

More specifically, and advantageously, a first object of the invention relates to an adjustable stretcher adapted to hold a subject lying in a supine position comprising:
- a plurality of supporting modules adapted to support one or more body regions of the subject, each of the supporting modules being connected to at least another supporting module through one or more connections, and wherein the connections are further adapted to modify the relative position between the supporting modules linearly and/or angularly; and
- an opening in a craniocaudal direction in relation to the position of the subject lying in the supine position, wherein the opening is adapted with a holder comprising means for receiving a spinal region treatment device and to position said spinal region treatment device with respect to the stretcher.

The described stretcher allows for keeping the patient's position fixed with respect to the spinal region treatment device between different treatment sessions. Since the greatest variability in position is in the patient's posture, especially with vertebral rotations or lateral tilts, fixing the patient's position in a comfortable way represents a good solution for avoiding variability in the treatment. Moreover, the opening provided with the holder allows for a versatile use of the stretcher, in the sense that any spinal region treatment device can be directly applied to the patient's region of interest. For instance, ultrasound delivering systems, radiofrequency delivering system and electrodes for electrostimulation, among others, may be used.

In a preferred embodiment of the invention, the stretcher further comprises a guiding system, the guiding system comprising two guiding rails arranged longitudinally in the craniocaudal direction in relation to the position of the subject lying supine in the cubitus position, each of said guiding rails being arranged at each side of the opening of the stretcher. Furthermore, the holder further comprises displacement means adapted to be inserted into the guiding rails of the guiding system such that the holder can be displaced in a longitudinal direction within the opening of the stretcher and the displacement means comprise locking means adapted to fix the position of the holder within the guiding rails of the guiding system.

In this way, the holder, and, therefore, the treatment device, can be displaced along the hole spinal region from the cervical to the lumbar area, allowing for treating any zone therein. This movement may be facilitated by incorporating a telescopic knob mechanism integrated within the guiding system, enabling precise and controlled adjustment of the spinal region treatment device position along the craniocaudal direction.

In another preferred embodiment of the invention, the stretcher comprises a supporting element, a backrest and a knee rest, and:
- the backrest and the knee rest's positions with respect to the supporting element are adjustable by means of adjusting rails comprised by the supporting element;
- the angle formed between the backrest and the supporting element is adjustable by means of a hinge actioned by a knob;
- the backrest comprises two boards arranged in a V-shape attached to a central canal for spine support; and
- the angle formed between the two boards can be graduated by means of hinges actioned by knobs.

This design considers the variability in the size of the patients including the proportions of the head, trunk, and legs. For this purpose, in addition to being hinged and adjustable to the shape of the spine, it is constructed to be able to vary its size and proportion to adapt to the high variability of human sizes. Furthermore, the design of the backrest is conceived such that the rotation movements on the craniocaudal axis are avoided, and the spine is placed at the vertex of the V, opening the back, and exposing it to the stimulation device.

In another preferred embodiment of the invention, the adjusting rails and each of the knobs comprise one or more scale rules corresponding to centimetric or goniometric values marking the positions of the backrest and knee rest with respect to the supporting piece, the angle between the backrest and the supporting piece and the angle between the two boards. This has the advantage that, once the subject is properly positioned on the stretcher for a particular treatment, the exact position of the various elements of the stretcher can be recorded for subsequent treatment sessions, thus avoiding the need to calibrate or adjust the stretcher in each session.

This is particularly advantageous given the typically low accuracy in the identification of anatomical landmarks. By using the stretcher of the invention as described in any of the preceding embodiments, once a reference point is identified within the spinal cord of the subject, since the exact position of the subject within the stretcher can be recorded, there is no need for re-identification of said anatomical landmark in posterior treatment sessions. This significantly reduces the possibility of anatomical landmark misidentification.

A second object of the invention relates to an external system for non-invasive neurostimulation of the spinal cord of a subject comprising an adjustable stretcher according to any of the previously described embodiments, and wherein:
- the spinal region treatment device comprises at least one focused ultrasound, FUS, transducer arranged at the holder, the FUS transducer array comprising a concave shape and being adapted to contact the skin of the subject and to deliver ultrasound waves, and arranged in such a way that, when the subject is held lying in the supine position on the stretcher, the curvature radius direction of the FUS transducer array is substantially perpendicular to a vertebral lamina of the subject and such that the focal point of the FUS transducer array is comprised within a target area comprised by the spinal cord of the subject; and
- a control unit connected to the at least one FUS transducer array, comprising a power source and processing and control means adapted to control the delivery of the ultrasound waves.

This system allows for non-invasive neurostimulation of the spinal cord for promoting neurorepair in patients with spinal cord diseases (spinal cord injury, MS, amyotrophic lateral sclerosis (ALS), stroke, tumors or others). The system has been designed and optimized for the specific anatomy of the human spinal cord considering the positioning of the subject during the sessions, enabling online but also, and more interestingly, off-line MRI scanning for positioning the device with respect to the vertebrae and spinal cord landmarks, as will be described further on. Most importantly, the design's system allows for targeting the spinal cord through the posterior lamina of the vertebrae, which is the thinnest bone structure in the vertebrae and thus implies less effects such as attenuation or aberration of the sound waves. Other systems attempt to reach the spinal cord though the inter-vertebrate foramen or between two contiguous spinous processes. However, such spaces are so small that irremediably the FUS beam will contact the bone and will be dispersed. Second, with aging, the inter-vertebrate space decreases and becomes highly heterogenous, hindering such approach in patients.

In a preferred embodiment of the invention, the spinal region treatment device (10) comprises at least two focused ultrasound, FUS, transducer arrays (17) arranged at the holder (9), the FUS transducer arrays (17) comprising a concave shape and being adapted to contact the skin of the subject and to deliver ultrasound waves, and arranged in such a way that, when the subject is held lying in the supine position on the stretcher (1), the curvature radius (27) direction of each transducer is substantially perpendicular to a vertebral lamina (19) of the subject and such that the focal points (20) of the two FUS transducer arrays (17) intersect in a target area comprised by the spinal cord of the subject. In this embodiment, the control unit (18) is connected to the at least two FUS transducer arrays (17).

This configuration is especially advantageous, since, on top of the above commented benefits regarding the one FUS transducer array approach, the spatial resolution of the FUS-delivering device is increased by the intersection of the two FUS beams at the corresponding focal points of the two FUS transducer arrays.

In another preferred embodiment of the invention, the ultrasound waves delivered by the system comprise low-intensity FUS, LIFUS, with an intensity comprised between 0.01 W/cm² and 2 W/cm²; maximum pressure values comprised between 0.1 MPa and 1 MPa and mechanical index values smaller than 1.9. These values target neurostimulation of the spinal cord as opposed to systems aimed at BSCBO. They also ensure the safety of the subject in clinical terms.

In another preferred embodiment of the invention, each of the FUS transducer arrays comprises:
- a plurality of piezoelectric elements arranged within the transducer without overlapping with one another;
- a diameter between 10mm and 100 mm; and/or
- a curvature radius between 10mm and 120 mm.
Preferably, the number of piezoelectric elements is fixed to 32, since this corresponds to the minimum number for which there is no target area of maximal pressure loss and ensures the correct delivery of the previously mentioned intensity and pressure values. This translates into maintaining the transducer as small as possible, first to fit in the stretcher and second to accommodate properly all kinds of anatomical differences that could appear between patients.

In another preferred embodiment of the invention, the angle formed by the directions of the radii of curvature of the two FUS transducer arrays is larger than 40° and smaller than 80° and/or the distance between the curvature center of each transducer and its corresponding focal point is between 40 mm and 80 mm.

In another preferred embodiment of the invention, the delivered ultrasound waves comprise a fundamental frequency comprised between 100 kHz and 500 kHz.

All these characteristics ensure a sufficiently small target area (high spatial resolution), corresponding to the intersection of the focal points of the transducers, and a relatively large intensity or maximal pressure within the parameters of the LIFUS. The fact that the angle formed by the curvature radiuses of the two transducers is set between 40° and 80° allows for each curvature radius to be perpendicular to the vertebrae' laminae. Moreover, the crossing the two beams improves considerably the spatial resolution.

In another preferred embodiment of the invention, the holder comprises an arc-shaped structure that comprises:
- one or more fixing elements, each adapted to fix the position and orientation of a FUS transducer array within the arc-shaped structure; and
- an arc-shaped rail arranged along the arc-shaped structure adapted to hold the fixing elements within said arc-shaped structure;
and wherein the fixing elements are adapted to be released and tightened, such that both the displacement of the FUS transducer arrays along the arc-shaped rail and the relative orientation of the transducers can be modified from at least a first position to a second position.

Advantageously, in another embodiment of the invention, the holder further comprises pivoting means adapted to rotate the arc-shaped structure in the roll, yaw, and pitch degrees of freedom.

With this configuration of the holder, the relative position and orientation of the transducer/s between one another in case there are more than one and with respect to the subject can be adjusted in a very precise way, adapting to all possible variabilities of said position and orientation depending on the requirements of the treatment and of the patient. This is especially advantageous in view of the high spatial resolution provided by the FUS.

In another preferred embodiment of the invention, the control unit comprise hardware and/or software means adapted to:
- process one or more MRI and/or CT images of the subject and segment therein the spinal cord by means of a segmentation algorithm;
- select, according to the previous spinal cord segmentation, one or more stimulation points located within the spinal cord of the subject;
- segment the laminae of the subject comprised by the MRI and/or CT images that correspond to the vertebrae surrounding the selected stimulation points by means of a segmentation algorithm and calculate the normal vector to the surface of said laminae;
- provide, according to the previous normal vectors, an optimal position of the FUS transducer array/s with respect to the stretcher, and, therefore, with respect to the the patient's back, for each of the selected stimulation points so that the delivery of the FUS waves is maximized;
- calculate, within the MRI and/or CT images, acoustic units, bone absorption, tissue density, speed of the acoustic waves throughout different structures comprised by said images and/or standing waves through the spinal canal; and/or
- determine, one or more spinal cord stimulation parameters including piezoelectric element phases for electronically steering the beam focus and/or aberration control signals;
and wherein the control means are adapted to receive the one or more spinal cord stimulation parameters and to provide instructions to the FUS transducer array/s such that said FUS transducer array/s deliver ultrasound waves as a function of said one or more spinal cord stimulation parameters.

Therefore, the control unit is adapted to provide an optimal position and orientation of the transducer/s with respect to the stretcher (or, equivalently, with respect to the subject lying on said stretcher in a fixed position) and optimal spinal cord stimulation parameters related to the characterization of the delivered ultrasound waves. This optimizes greatly the efficacy of the treatment and targets specifically the desired areas to be stimulated. Furthermore, the processing of the MRI and/or CT images can be carried out online, i.e., introducing the patient and the system into an MRI machine or a CT scan, but also off-line, after the images have been obtained independently. This later case avoids size and shape restrictions of the system that may be uncomfortable for the patient (since it does not need to be fitted into a machine).

It is to be noted that a system according to any of the previously described embodiments but in which either the stretcher is not present or another type of support for the subject and/or the FUS delivering device is provided is also possible and advantageous. The FUS-delivering device and the control unit can operate independently from the stretcher in order to efficiently stimulate the spinal cord of a subject, provided that said FUS-delivering device is properly arranged as described above (i.e., with the curvature radius direction of the FUS transducer arrays substantially perpendicular to a vertebral lamina of the subject and such that the focal point of the FUS transducer arrays are comprised within a target area comprised by the spinal cord of the subject).

Finally, a third object of the invention refers to a method for optimizing the configuration of a system for non-invasive neurostimulation of the spinal cord of a subject according to any of the previously described embodiments, wherein the subject is lying supine one the adjustable stretcher. Said method comprises performing the following steps:
a) adjusting the position and orientation of the supporting modules of the stretcher according to the subject's body and such that the spine of the subject is arranged within the opening of the stretcher;
b) receiving one or more MRI and or CT images of the spinal cord of a subject;
c) processing the images obtained in step b) by using the processing means and:
   - identifying a set of anatomical landmarks within the spinal cord of the subject;
   - identifying one or more stimulation points within the spinal cord of the subject;
   - identifying the laminae of the subject comprised by the MRI and/or CT images that correspond to the vertebrae surrounding the selected stimulation points;
d) placing and orienting, according to the anatomical landmarks obtained in step c), the FUS transducer array/s such that that the curvature radius direction of each transducer is perpendicular to a lamina corresponding to the vertebra surrounding an identified stimulation point and such that the focal points of each FUS transducer array substantially coincides with said identified stimulation point; and
f) recording the position and orientation of the of the supporting modules, the set of anatomical landmarks, the one or more stimulation points and the position and orientation of the FUS transducer array/s for future configurations of the system.

Advantageously, this method provides a precise way of arranging and configuring the described system by placing and orienting its different elements such that the effect of the ultrasound waves is maximized in the desired stimulation area, always within the corresponding safety parameters. Furthermore, all the resulting parameters, positions, landmarks, etc. are recorded for future treatment sessions so that the system does not need to be recalibrated for the same subject. Again, it is to be noted that the reception and processing of the MRI and/or CT images can be carried out online, i.e., introducing the patient and the system into an MRI machine or a CT scan, but also off-line, after the images have been obtained independently of the other steps of the method.

In another preferred embodiment of the invention, after step c) and before step f), the previously described method comprises performing the following steps by using the processing means:
- calculating, within the MRI and/or CT images: acoustic units, bone absorption, tissue density, speed of the acoustic waves throughout different structures comprised by said images and/or standing waves through the spinal canal; and
- determining, from the previous calculation, one or more spinal cord stimulation parameters including piezoelectric element phases for electronically steering the beam focus and/or aberration control signals.

This information is especially useful for configuring the FUS transducer array/s so that the stimulation effect induced by the ultrasound waves is maximized in the desired stimulation points.

In another preferred embodiment of the invention, after step c) and before step f), the previously described method comprises performing the following steps by using the processing means:
- simulating, based on the previous determination, on the measurements of the subject received in step a), on the anatomical landmarks, stimulation points and laminae identified in step c), and on the position and orientation of the FUS transducer array/s in step d) the stimulation effect of the delivery of FUS waves;
- fine-tuning, based on the previous simulation, the values of the one or more spinal cord stimulation parameters; and/or
- sending the fined-tuned values of the one or more spinal cord stimulation parameters to the control means and recording the fined-tuned values of the one or more spinal cord stimulation parameters for future configurations of the system.

Performing simulations of the effect induced by the ultrasound waves before actually delivering them to an in vivo patient may save time and costs, since no trial-and-error treatments have to be carried out in order to fine tune the system for an efficient treatment. Furthermore, this can be done in a personalized way, i.e., for each patient individually. The simulations may be performed by artificial intelligence, machine learning and/or computer vision techniques, such as Generative algorithms, among others.

In summary, the proposed invention provides an apparatus that allows for an optimal positioning of a spinal region treatment device (especially if said device is FUS-based) with respect to the subject to be treated, a system for non-invasive and efficient neurostimulation of the spinal cord and a method for optimizing the configuration of said system.

Unless otherwise defined, all terms (including technical and scientific terms) used herein are to be interpreted as is customary in the art. It will be further understood that terms in common usage should also be interpreted as is customary in the relevant art and not in an idealized or overly formal sense unless expressly so defined herein.

Within the context of the present invention, the term "concave" is to be interpreted as a hollowed or rounded inward, i.e., a bowl-shaped structure, preferably defined by a diameter and a radius of curvature.

### BRIEF DESCRIPTION OF DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 shows a schematic representation of an adjustable stretcher according to an embodiment of the present invention.
Figure 2 shows a schematic representation of an external system for non-invasive stimulation of the spinal cord based on FUS according to an embodiment of the present invention.
Figure 3 shows a schematic representation of two views (upper and lateral) of a holder adapted to receive two ultrasound transducers according to an embodiment of the present invention.
Figure 4 shows a configuration of a two-FUS transducer arrays approach according to an embodiment of the present invention. Figure 4A shows a representation of the piezoelectric elements comprised by the transducers and a possible orientation of said transducers in a 3D space. Figure 4B shows a specific arrangement of the transducers with respect to the vertebral column and lamina appearing in an MRI image.
Figure 5 shows six anatomical measurements and landmarks performed in a sagittal view of the vertebral column performed over CT images, with a specific example of the L1 vertebra.
Figure 6 shows a density map computed from a simulation domain constructed from CT images containing a density volume along with two FUS transducer arrays according to an embodiment of the present invention placed therein for visual aid.
Figure 7 shows a density map computed from a simulation domain constructed from CT images containing a density volume.
Figure 8 shows the pressure distribution delivered by two FUS transducer arrays according to an embodiment of the present invention within a simulation domain for two different frequencies of the FUS waves: 200 kHz (Figure 8A) and 300 kHz (Figure 8B).

### NUMERIC REFERENCES USED IN THE DRAWINGS

| | |
|---|---|
| 1 | Adjustable stretcher |
| 2 | Supporting modules |
| 3 | Supporting element |
| 4 | Backrest |
| 5 | Knee rest |
| 6 | Adjusting rails |
| 7 | Boards |
| 8 | Opening |
| 9 | Holder |
| 10 | Treatment device |
| 11 | Guiding system |
| 11' | Guiding rails |
| 12 | Displacement means |
| 13 | Locking means |
| 14 | Scale rules |
| 15 | Cushions |
| 16 | System |
| 17 | FUS transducer arrays |
| 18 | Control unit |
| 19 | (Vertebral) lamina |
| 20 | Focal point (of the FUS transducer arrays) |
| 21 | Stimulation point |
| 22 | Arc-shaped structure |
| 23 | Fixing elements |
| 24 | Arc-shaped rail |
| 25 | Pivoting means |
| 26 | Diameter (of the FUS transducer arrays) |
| 27 | Curvature radius (of the FUS transducer arrays) |
| 28 | Center of curvature (of the FUS transducer arrays) |

### DETAILED DESCRIPTION OF THE INVENTION

The example embodiments are described in sufficient detail to enable those of ordinary skill in the art to embody and implement the devices, systems and processes herein described. Embodiments can be provided in many alternate forms and should not be construed as limited to the examples set forth herein.

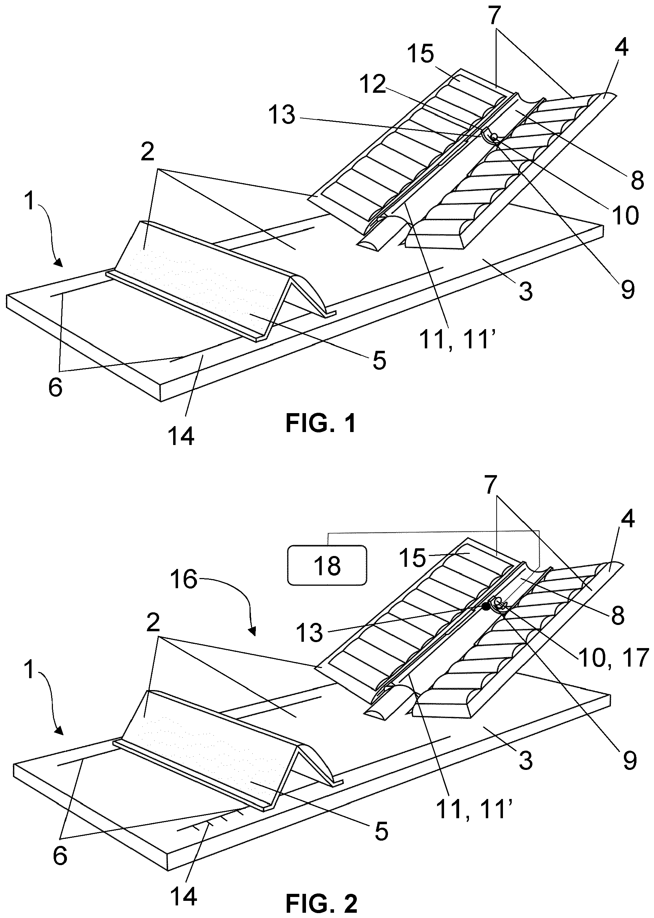

Accordingly, while embodiments can be modified in various ways and take on various alternative forms, specific embodiments thereof are shown in the drawings and described in detail below as examples. There is no intent to limit to the particular forms disclosed. On the contrary, all modifications, equivalents, and alternatives falling within the scope of the appended claims should be included. Elements of the example embodiments are consistently denoted by the same reference numerals throughout the drawings and detailed description where appropriate. Some preferred embodiments of the invention, shown in Figs. 1-7, will be now described for illustrative, but not limiting purposes.

Figure 1 shows a schematic representation of an adjustable stretcher (1) adapted to hold a subject lying in a supine position according to an embodiment of the present invention. The stretcher (1) comprises a plurality of supporting modules (2), each adapted to hold one or more body regions of a subject and connected to at least another supporting module (2).

More specifically, Figure 1 shows an embodiment of the invention in which the stretcher (1) comprises three supporting modules (2) corresponding to a supporting element (3), a backrest (4) and a knee rest (5). The knee rest (5) is connected to the supporting element (3) by means of adjusting rails (6) comprised by said supporting element (3). This allows for adapting the dimensions of the stretcher (1) to those of the patient lying on it, so that the back and the knees are correctly placed onto the backrest (4) and the knee rest (5), respectively. Moreover, the backrest (4) is connected to the supporting element (3) by means of a hinge that may be actioned by a knob, so that the angle formed between the backrest (4) and the supporting element (3) can be adjusted. This enables a correct placement of the subject depending on the treatment to be delivered, as well as ensuring their comfort.

As seen in Figure 1, the backrest (4) comprises two boards (7) arranged in a V-shape attached to a central canal for spine support. This leaves an opening (8) in the craniocaudal direction in relation to the position of the subject lying in the supine position in which the spinal region of the subject may be placed. Advantageously, the angle formed between the two boards (7) of the backrest (4) can be graduated by means of hinges, preferably actioned by knobs.

The opening (8) comprised by the stretcher (1) is adapted with a holder (9) comprising means for receiving a spinal region treatment device (10) such as an ultrasound transducer, a radiofrequency transducer, or an electrode array for electric stimulation, among others. As seen in Figure 1, the stretcher (1) further comprises a guiding system (11) comprising two guiding rails (11') arranged longitudinally in the craniocaudal direction, each of said guiding rails (11') being arranged at each side of the opening (8) of the stretcher. On the other hand, the holder (9) further comprises displacement means (12) adapted to be inserted into the guiding rails (11') of the guiding system (11) such that the holder (9) can be displaced in a longitudinal direction within the opening (8) of the stretcher (1). Advantageously, the displacement means (12) comprise locking means (13) so that the position of the holder (9) within the guiding system (11) can be fixed. The possibility of moving the holder (9), and therefore a treatment device (10), in the longitudinal direction of the opening (8) allows for treating the whole spinal region (from cervical to sacral) without laborious reconfigurations of the stretcher (1). Thus, in the same treatment session, the position of the treatment device (10) can be fixed at a first high with respect to the vertebral column of the subject and then moved and fixed to a second high for treating a different region within the spinal region of the subject.

Advantageously, the stretcher (1) comprises a set of scale rules (14), corresponding to each rail and knob, marking centimetric and goniometric values, respectively. These serve to denote the positions of the backrest (4) and knee rest (5) with respect to the supporting element (3), the angle between the backrest (4) and the supporting element (3) and the angle between the two boards (7) of the backrest (4), as well as the position of the holder (9) within the opening (8). This way, a particular configuration of the stretcher's (1) elements can be registered for future treatment sessions, without the need for recalibrating the stretcher (1) for the same subject in each session.

The supporting modules (2) of the stretcher (1) may be preferably made of high-density polyethylene (HDPE) due to its excellent strength-to-weight ratio, impact resistance, and durability. This material is lightweight yet provides sufficient rigidity and support for patient transportation. HDPE is also resistant to chemicals, moisture, and UV radiation. Alternatively, polypropylene (PP) may be used, since it offers good mechanical strength, chemical resistance, and thermal stability being also lightweighted. Optionally, certain parts of the stretcher (1) may comprise cushions (15) made of a cushioning material to increase the subject's comfort. The cushioning material may be, preferably, a polyurethane foam of different densities and firmness levels, allowing for customization based on the desired level of support.

The described stretcher (1) is particularly advantageous when included in a system (16) for spinal treatment. Figure 2 shows such a system (16) according to an embodiment of the present invention. In particular, said system (16) is aimed at stimulating the spinal cord of a subject in a non-invasive way taking advantage of the benefits of focus ultrasound technology.

The system (16) shown in Figure 2 comprises three main elements: a stretcher (1) as described previously, two focused ultrasound, FUS, transducer arrays (17) and a control unit (18). In the system (16), the FUS transducer arrays (17) correspond to the spinal region treatment device (10) and are thus mounted on the holder (9) comprised by the stretcher's opening (8).

Advantageously, the FUS transducer arrays (17) comprise a concave shape and are adapted to contact the skin of the subject and to deliver ultrasound waves. This process may involve the use of contact gels or other contact materials for improving transmission of the ultrasound waves. The FUS transducer arrays (17) are arranged in such a way that, when the subject is held lying in the supine position on the stretcher (1), the curvature radius direction of each FUS transducer array (17) is substantially perpendicular to a vertebral lamina (19) of the subject and such that the focal points (20) of the two FUS transducer arrays (17) intersect in a target area comprised by the spinal cord of the subject. In this way, the ultrasound beams pass through the thinnest possible bone area (that corresponding to the lamina (19)) before reaching the spinal cord, which reduces interference and possible attenuation of the beams. Moreover, the use of two FUS transducer arrays (17) allows for the effects of the ultrasound beams coming from each FUS transducer array (17) to be superimposed at the desired stimulation point (21), increasing their spatial resolution.

The control unit (18) is connected to the FUS transducer arrays (17) and comprises a power source, adapted to provide the necessary energy to deliver the ultrasound waves, and processing and control means adapted to control the delivery of the ultrasound waves. This control may involve from switching the power source on and off to a more precise control of various parameters (frequency, amplitude, phase, among others) associated with the ultrasound waves. A firmware system for the electronics and an API may be included for controlling the system.

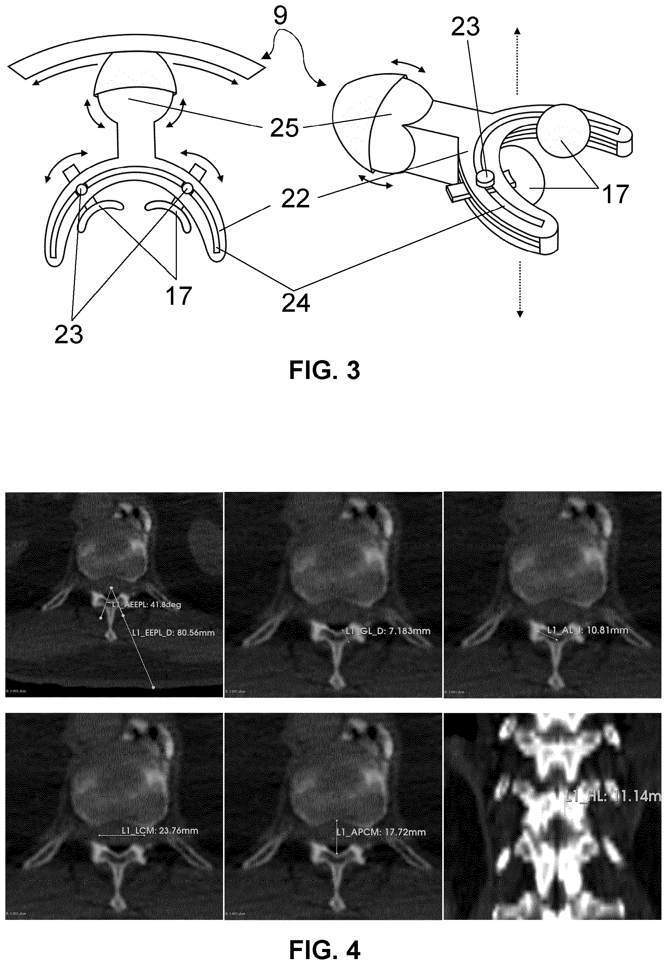

Advantageously, the FUS transducer arrays (17) can be arranged at the holder (9) in a specific way that allows for a great variability in their position and orientation. Figure 3 shows such a configuration in which the holder (9) comprises an arc-shaped structure (22) that comprises, in turn, two fixing elements (23), each adapted to fix the position and orientation of a FUS transducer array (17) within the arc-shaped structure (22). In addition, the arc-shaped structure comprises an arc-shaped rail (24), arranged along the arc, which is adapted to hold the fixing elements (23).

The fixing elements (23) are adapted to be released and tightened, such that both the displacement of the FUS transducer arrays (17) along the arc-shaped rail (24) and the relative orientation of the FUS transducer arrays (17) can be modified. It is to be noted that this can be done independently for each of the FUS transducer arrays (17), enabling a large range of possible spatial configurations. A screw with a nut may serve as fixing elements (23), as they can be tightened and released when inserted in the arc-shaped rail (24), holding the FUS transducer arrays (17). However, any other standard fixing element known in the art could be employed, provided that it comprised the described features.

In Figure 3 it can also be observed that the holder (9) further comprises pivoting means (25) adapted to rotate the arc-shaped structure (22) in the roll, yaw, and pitch degrees of freedom. This, in combination with the previously described arc-shaped structure (22) and related features, allows for positioning and orienting the FUS transducer arrays (17) in any direction in space and in any orientation, so that they can be adapted to the specific needs of the desired treatment and the subject to be treated. Besides, it allows for targeting very accurately the desired stimulation point (21), which is especially relevant for the present system due to the high spatial resolution of the FUS technique.

Furthermore, as seen in Figures 1 and 2, the opening (9) of the stretcher (1) in which the holder (9) is arranged may comprise a curved surface, i.e., the opening may comprise a hollow half-cylinder shape. In this embodiment, the holder (9) may comprise displacement means so that, beyond the possibility of being moved longitudinally along the opening (8), it may also be moved transversally, along the half-cylinder's perimeter, for any given position in the longitudinal direction.

Beyond the spatial and structural configuration of the system (16) of the invention, the design of the FUS transducer arrays (17) and the selection of the FUS waves' parameters are of great relevance for achieving maximal efficiency of the spinal cord stimulation. With this aim, a study has been conducted in order to determine the optimal technical features of the FUS transducer arrays (17) and of the FUS waves stimulation parameters.

First, an anatomical analysis of the spinal region (vertebral column and spinal cord) of 5 different patients has been carried out. Measurements of 3 vertebrae (L1, T10, and C6) from each patient were taken, amounting to a total of 9 measurements and one coefficient for each vertebra. Back-spine through lamina (BSTL) from left (BSTL_L) and right sides (BSTL_R), and the corresponding angle (ABSTL) related measurements are used to study how the location of the FUS transducer arrays (17) should be in relation to the vertebrae and the back. Thickness of the lamina (TL), width of the lamina (WL), and high of the lamina (HL) give information about the structure, shape, and size of the lamina (19) which is the target zone to traverse the bone. The area of the spinal canal (ASC), antero-posterior spinal canal (APSC), lateral spinal canal (LSC), and LSC/APSC ratio give information about the size of the spinal canal which contains the spinal cord that is the structure of interest for the stimulation. This study was also aimed at ensuring that differences among patients will not be relevant in the present case, in order to avoid incorrect uses or malfunctions of the device due to that variability.

Figure 4 shows an example of how the measurements have been carried out using CT images of the neck, abdomen, and pelvis of a particular patient. The vertebra used in this case is L1, representative of the lumbar area. All other vertebrae of all the patients are measured as shown in Figure 4.

With the collected anatomical information, a number of computer simulations regarding FUS transducer array (17) characteristics has been performed.

First, regarding the number of piezoelectric elements comprised by each FUS transducer array (17), simulations with 16, 32 and 64 elements were run, pointing at 32 as the optimal number of piezoelectric elements since it corresponds to the minimum number for which there is no target area of maximal pressure loss. This translates into maintaining the FUS transducer arrays (17) as small as possible, first to fit them in the holder (9) of the stretcher (1) and second to accommodate properly all kinds of anatomical differences that could appear between patients.

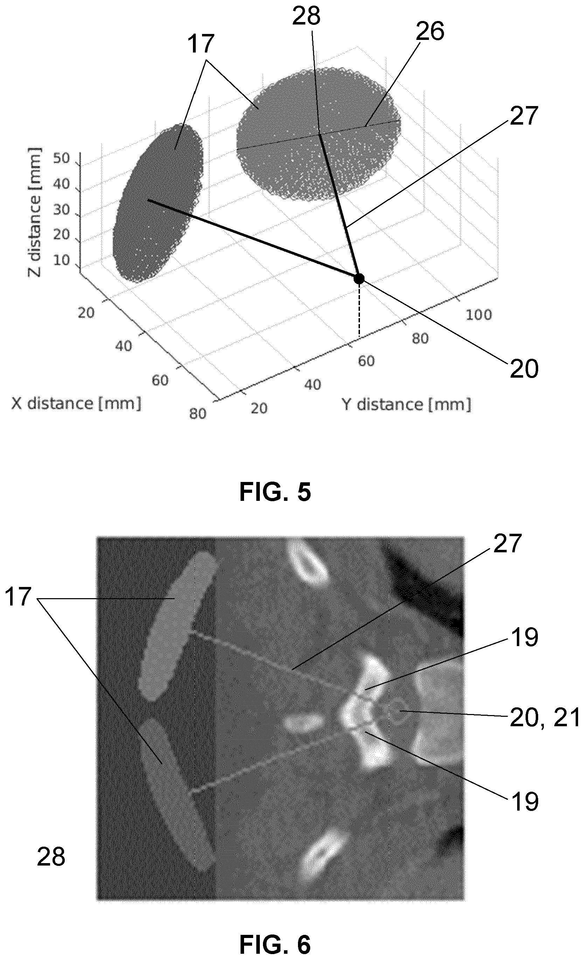

Then, the dimensional and geometrical characteristics of the FUS transducer arrays (17) were analyzed. A double FUS transducer array (17) approach with two concave transducer arrays (17) of 32 piezoelectric elements was taken. Each of the FUS transducer arrays (17) had a diameter (26) of 49 mm and a curvature radius (27) of 60 mm, as seen in Figure 5, where the double FUS transducer array (17) is shown as it is positioned inside the simulation domain. An angle of 60° between the curvature radii (27) of the two FUS transducer arrays (17), was set. The selection of the 60° angle was taken based on the results of the ABSTL measurements. The focal point (20) is defined at [80,64,64] mm in the x,y,z axis, and represented as the intersection of the two lines, each corresponding to the curvature radius (27) of the FUS transducer arrays (17). The focal point (20) is thus at a distance of 80 mm from the plane comprising the centers of the rear surface or centers of curvature (28) of the FUS transducer arrays (17).

As seen in Figure 5, the curvature radius (27) of the FUS transducer arrays (17) determines where the natural focal point (20) is positioned. Precisely, the optimal focusing point (20) for a given radius R will be at F = R + B, where B is the position of the center of curvature (28). The focal point (20) can then be modified by applying delays to the stimulation of the piezoelectric elements, which leads to an alteration in the beamform of the FUS transducer arrays (17). Therefore, taking into account that the depths at which the spinal cord (and thus the stimulation point (21)) is located vary between the three spinal column regions (lumbar, thoracic, and cervical), the curvature radius (27) has to be carefully selected.

A mean distance to the spinal cord of 60 mm was obtained from BSTL measurements, and from a standard deviation of 15 mm, it was assumed that the focal point (20) will have to be modified around 20 mm backwards and forwards. Therefore, in an embodiment of the present invention, a curvature radius (27) between 40 mm and 80 mm is selected, leading to focal points (20) positioned at distances between 40 and 80 mm from the plane comprising the centers of curvature (28) of the FUS transducer arrays (17). This ensures a complete coverage of the entire possible range of distances to the spinal cord and thus to the stimulation point (21). Accordingly, the selected values for the diameter (26) of the FUS transducer arrays (17) are selected to be comprised between 40 mm and 60 mm. This ensures that, for the minimal angle of aperture between radii, the transducers will not overlap.

In the embodiment of the invention shown in Figure 5, both FUS transducer arrays (17) are identical and point towards the same focal point (20) in order to achieve a crossing of their beams to reduce the stimulation area. It is to be noted that this represents a non-limiting embodiment, since, depending on the stimulation requirements, the FUS transducer arrays (17) may be different from one another, may comprise an irregular shape, etc.

Nonetheless, in Figure 5, the focal point (20) is placed along the radius line of the FUS transducer arrays (17). The angle between said lines influences the beam characteristics and thus has to be carefully set in order to have an optimal beam. The opening angle between the FUS transducer arrays (17), i.e., the angle formed between their curvature radii (27), has been identified as optimal in the range of 40°- 80°, and preferably at 60°. This opening angle is mainly influenced by the angle formed by the normal vectors of the surface of the laminas, which has been found to be 60° on average.

Beyond the FUS transducer array (17) characteristics, a computer analysis based on different simulations regarding the FUS waves' parameters for stimulation has been performed.

This analysis, together with the standard safety guidelines in the delivery of ultrasound waves, has shown that the optimal values for the different parameters defining the ultrasound waves with the aim of accurately and safely stimulate the spinal cord of a human being are the following:
- intensity comprised between 0.1 W/cm² and 1 W/cm²;
- maximum pressure values comprised between 0.1 MPa and 1 MPa;
- mechanical index values smaller than 1.9;
- fundamental frequency comprised between 100 kHz and 500 kHz.
These values differ from other values used in spinal cord treatments aimed at a different result, such as in BSCBO techniques.

All simulations have been formed taking into account the different structures and tissues that the FUS waves may encounter in their path from the FUS transducer arrays (17) to the stimulation point (21). Introducing a bone medium creates a deformation of the beam and amplitude attenuation in the region of density change. Some of the effects observed are, for example, large intensity lobes produced inside the wall of the bone, grating lobes along the medium, sharpening of the beam area, focus loss, and reverberations. Thus, it is important to take these considerations into account when the simulations are carried out.

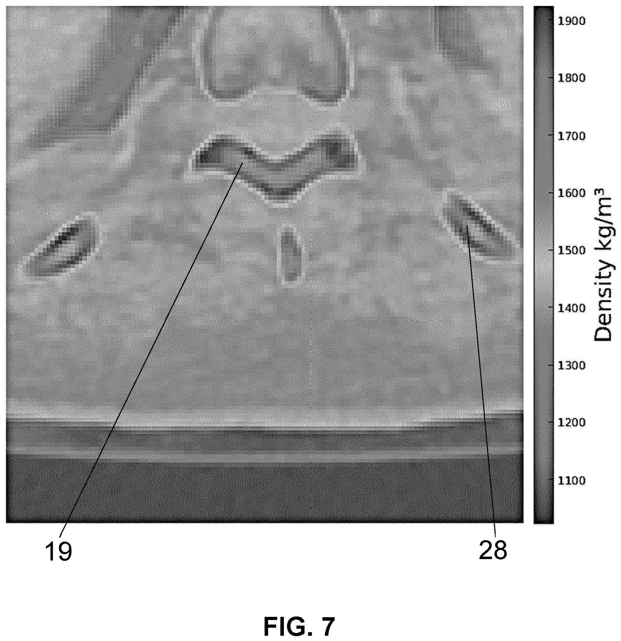

Figures 6 and 7 show tissue maps extracted from CT images from which the simulation results for the mockup bone and other structures have been conducted. In Figure 6 the FUS transducer arrays (17), together with their focal points (20) intersecting at the stimulation point (21) and their focal lines being perpendicular to the lamina (19) wall are represented for visual aid. In the simulations, the bone medium density is set to 1900 kg/m³ and the rest to 1000 kg/m³. Figure 7 shows the density map within the simulation domain, directly extracted from CT images, from which simulations containing a density volume with the FUS transducer arrays (17) placed inside a gel-like matrix to simulate the interface between the FUS transducer arrays (17) and the tissue are carried out.

Simulations of the effects of the delivery of the FUS transducer arrays (17) to a subject lying supine in the stretcher (1) according to the present invention were carried out using CT images and the designed FUS transducer arrays (17). From CT images, a volume containing the region of interest of the two vertebrae T10 and T9 was extracted. As shown is Figure 6, the FUS transducer arrays (17) were placed inside the medium setting their focal points (20) at the center of the spinal cord with the focus lines perpendicular to the lamina (19) wall. The FUS transducer arrays (17) were assumed to be inside a gel-like material with a density similar to that of the tissue surrounding the bones (1300 kg/m^3). The image units were transformed to a density map as shown in Figure 7, where the maximum value of density was set to that of cortical bone, 1900 kg/m^3, and the minimum to water density,1000 kg/m^3.

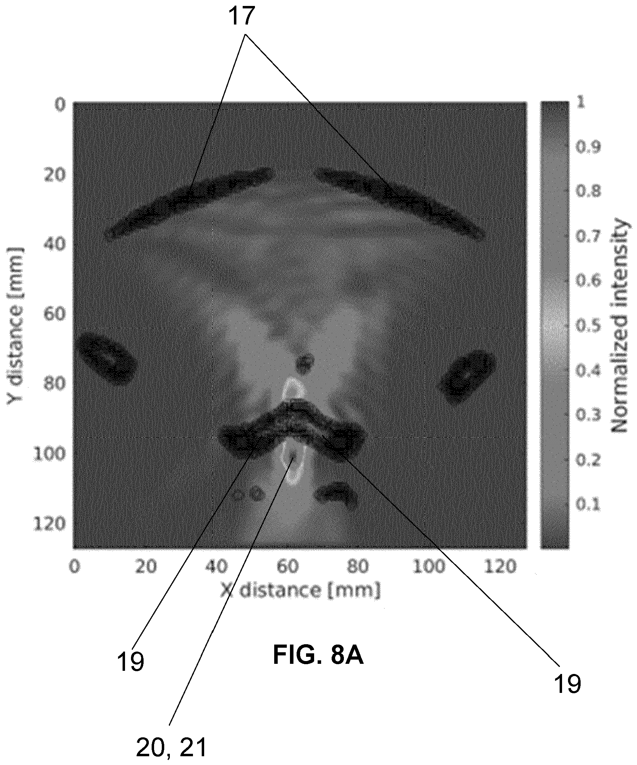

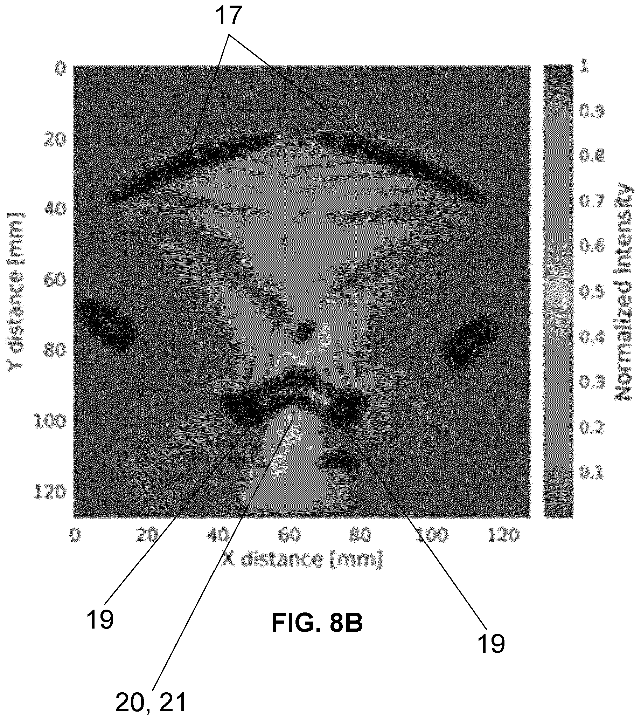

Simulation results are shown in Figure 8 for two different frequencies: 200 kHz in Figure 8A and 300 kHz in Figure 8B; where the penetration capabilities of the pressure beam are depicted.

All the parameters associated with the configuration of the FUS transducer arrays (17) and the ultrasound waves, as well as the position and orientation of the FUS transducer arrays (17) with respect to the stretcher (1) and, therefore, with respect to the subject, can, however, be determined for each patient or treatment.

Advantageously, the processing means of the control unit (18) comprise hardware and/or software means adapted to:
- process one or more MRI and/or CT images of the subject and segment therein the spinal cord by means of a segmentation algorithm, which may be done online or offline, i.e., with the patient and the system inside (online) or outside (offline) the MRI machine and/or the CT scan;
- select, according to the previous spinal cord segmentation one or more stimulation points (21) located within the spinal cord of the subject;
- segment the laminae (19) of the subject comprised by the MRI and/or CT images that correspond to the vertebrae surrounding the selected stimulation points (21) by means of a segmentation algorithm and calculate the normal vector to the surface of said laminae (19);
- provide, according to the previous normal vectors, an optimal position of the FUS transducer arrays (17) with respect to the stretcher (1) for each of the selected stimulation points (21) so that the delivery of the FUS waves is maximized;
- calculate, within the MRI and/or CT images, acoustic units, bone absorption, tissue density, speed of the acoustic waves throughout different structures comprised by said images and/or standing waves through the spinal canal; and/or
- determine, one or more spinal cord stimulation parameters including piezoelectric element phases for electronically steering the beam focus and/or aberration control signals.

Besides, the control means may be adapted to receive the one or more spinal cord stimulation parameters and to provide instructions to the FUS transducer arrays (17) such that they deliver ultrasound waves calibrated with one or more spinal cord stimulation parameters.

The fact that the control unit (18) and its processing means are adapted to perform the above-mentioned tasks, allows for a precise configuration and calibration of the system (16) of the invention such that the spinal cord stimulation is maximized in each treatment and for each subject. Thus, the present invention also provides a method for optimizing the configuration of the described system.

Said method comprises performing the following steps:
a) adjusting the position and orientation of the supporting modules (2) of the adjustable stretcher (1) according to a subject's body lying supine on said adjustable stretcher (1), and such that the spine of the subject is arranged within the opening (8) of the stretcher (1);
b) receiving one or more MRI and or CT images of the spinal cord of a subject (either online or offline);
c) processing the images obtained in step b) by using the processing means (either online or offline) and:
   - identifying a set of anatomical landmarks within the spinal cord of the subject;
   - identifying one or more stimulation points (21) within the spinal cord of the subject;
   - identifying the laminae (19) of the subject comprised by the MRI and/or CT images that correspond to the vertebrae surrounding the selected stimulation points (21);
d) placing and orienting, according to the anatomical landmarks obtained in step c), the two FUS transducer arrays (17) such that the curvature radius direction of each FUS transducer arrays (17) is perpendicular to a lamina (19) corresponding to the vertebra surrounding an identified stimulation point (21) and such that the focal points (20) of the two FUS transducer arrays (17) intersect substantially in said identified stimulation point (21); and
f) recording the position and orientation of the of the supporting modules (2), the set of anatomical landmarks, the one or more stimulation points (21) and the position and orientation of the FUS transducer arrays (17) for future configurations of the system (16).

Steps b) and c) are performed by the processing means comprised by the control unit (18), and steps a), d) and f) may be performed manually by a technician of physician, or automatically by incorporating actuators into the design of the system (16).

Optionally, after step c) and before step f), the following actions can be advantageously performed:
- calculating, within the MRI and/or CT images: acoustic units, bone absorption, tissue density, speed of the acoustic waves throughout different structures comprised by said images and/or standing waves through the spinal canal; and
- determining, from the previous calculation, one or more spinal cord stimulation parameters including piezoelectric element phases for electronically steering the beam focus and/or aberration control signals.

After obtaining the one or more spinal cord stimulation parameters, they may be sent to the control means so that they can be provided to the FUS transducer arrays (17) in order for them to deliver ultrasound waves calibrated accordingly. Moreover, the values of the spinal cord stimulation parameters may be recorded for future treatment sessions.

Finally, beyond the simulations run initially to determine optimal features of the system (16), a more detailed, personalized simulation may be carried out for each patient and treatment. With this aim, after step c) and before step f), the following actions can be advantageously performed:
- simulating, based on the previous determination, on the measurements of the subject received in step a), on the anatomical landmarks, stimulation points (21) and laminae (19) identified in step c), and on the position and orientation of the FUS transducer arrays (17) in step d) the stimulation effect of the delivery of FUS waves;
- fine-tuning, based on the previous simulation, the values of the one or more spinal cord stimulation parameters; and/or
- sending the fined-tuned values of the one or more spinal cord stimulation parameters to the control means and recording the fined-tuned values of the one or more spinal cord stimulation parameters for future configurations of the system.

As seen in Figure 8, this procedure is particularly advantageous, since, in some scenarios (Figure 8A), the spinal cord stimulation parameters may be already optimal whereas in others they may need adjustments in order to maximize the efficiency of the treatment (Figure 8B). In Figure 8A, it can be seen that the pressure delivered by the FUS transducer arrays (17) is maximal at the stimulation point (21) comprised by the spinal cord of the subject, depicted as a darker set of points in the simulation. In the Figure 8B case, however, the maximum pressure point is not comprised by the spinal cord of the subject within that configuration.

Thus, the present invention provides an adjustable stretcher (1) that allows for an optimal positioning of a spinal region treatment device (especially if said device is FUS-based) with respect to the subject to be treated, a system (16) for non-invasive and efficient neurostimulation of the spinal cord and a method for optimizing the configuration of said system.

## Claims

1. Adjustable stretcher (1) adapted to hold a subject lying in a supine position **characterized in that** the adjustable stretcher (1) comprises:
- a plurality of supporting modules (2) adapted to support one or more body regions of the subject, each of the supporting modules (2) being connected to at least another supporting module (2) through one or more connections, and wherein the connections are further adapted to modify the relative position between the supporting modules (2) linearly and/or angularly; and
- an opening (8) in a craniocaudal direction in relation to the position of the subject lying in the supine position, wherein the opening (8) is adapted with a holder (9) comprising means for receiving a spinal region treatment device (10) and to position said spinal region treatment device (10) with respect to the stretcher (1).

2. Adjustable stretcher (1) according to the preceding claim, wherein:
- the stretcher (1) further comprises a guiding system (11), the guiding system (11) comprising two guiding rails (11') arranged longitudinally in the craniocaudal direction in relation to the position of the subject lying supine in the cubitus position, each of said guiding rails (11') being arranged at each side of the opening (8) of the stretcher (1);
- the holder (9) further comprises displacement means (12) adapted to be inserted into the guiding rails (11') of the guiding system (11) such that the holder (9) can be displaced in a longitudinal direction within the opening (8) of the stretcher (1); and
- the displacement means (12) comprise locking means (13) adapted to fix the position of the holder (9) within the guiding rails (11') of the guiding system (11).

3. Adjustable stretcher (1) according to any of the preceding claims, wherein the stretcher (1) comprises a supporting element (3), a backrest (4) and a knee rest (5), and wherein:
- the backrest (4) and the knee rest's (5) positions with respect to the supporting element (3) are adjustable by means of adjusting rails (6) comprised by the supporting element (3);
- the angle formed between the backrest (4) and the supporting element (3) is adjustable by means of a hinge actioned by a knob;
- the backrest (4) comprises two boards (7) arranged in a V-shape attached to a central canal for spine support; and
- the angle formed between the two boards (7) can be graduated by means of hinges actioned by knobs.

4. Adjustable stretcher (1) according to the preceding claim, wherein the adjusting rails (6), the guiding rails (11'), and each of the knobs comprise one or more scale rules (14) corresponding to centimetric or goniometric values marking the positions of the backrest (4) and knee rest (5) with respect to the supporting element (3), the angle between the backrest (4) and the supporting element (3), the angle between the two boards (7), and the position of the holder (9) within the guiding system (11).

5. External system (16) for non-invasive neurostimulation of the spinal cord of a subject comprising an adjustable stretcher (1) according to any of the preceding claims and **characterized in that**
- the spinal region treatment device (10) comprises at least one focused ultrasound, FUS, transducer array (17) arranged at the holder (9), the FUS transducer array (17) comprising a concave shape and being adapted to contact the skin of the subject and to deliver ultrasound waves, and arranged in such a way that, when the subject is held lying in the supine position on the stretcher (1), the curvature radius (27) direction of the FUS transducer array (17) is substantially perpendicular to a vertebral lamina (19) of the subject and such that the focal point (20) of the FUS transducer array (17) is comprised within a target area comprised by the spinal cord of the subject; and
- the system (16) further comprises a control unit (18) connected to the at least one FUS transducer array (17), comprising a power source and processing and control means adapted to control the delivery of the ultrasound waves.

6. External system (16) for non-invasive neurostimulation of the spinal cord of a subject comprising an adjustable stretcher (1) according to the preceding claim, wherein the spinal region treatment device (10) comprises at least two focused ultrasound, FUS, transducer arrays (17) arranged at the holder (9), the FUS transducer arrays (17) comprising a concave shape and being adapted to contact the skin of the subject and to deliver ultrasound waves, and arranged in such a way that, when the subject is held lying in the supine position on the stretcher (1), the curvature radius (27) direction of each transducer is substantially perpendicular to a vertebral lamina (19) of the subject and such that the focal points (20) of the two FUS transducer arrays (17) intersect in a target area comprised by the spinal cord of the subject;
and wherein the control unit (18) is connected to the at least two FUS transducer arrays (17).

7. System (16) according to any one of claims 5-6, wherein the ultrasound waves comprise low-intensity FUS, LIFUS, with an intensity comprised between 0.01 W/cm² and 2 W/cm²; maximum pressure values comprised between 0.1 MPa and 1 MPa, mechanical index values smaller than 1.9 and/or a fundamental frequency comprised between 100 kHz and 500 kHz.

8. System (16) according to any one of claims 5-7, wherein each of the FUS transducer arrays (17) comprise:
- a plurality of piezoelectric elements arranged within the transducer without overlapping with one another;
- a diameter (26) between 10 mm and 100 mm; and/or
- a curvature radius (27) between 10 mm and 120 mm.

9. System according to any one of claims 6-8, wherein:
- the angle formed by the directions of the curvature radii (27) of the two FUS transducer arrays (17) is larger than 40° and smaller than 80°; and/or
- the distance between the curvature center (28) of each FUS transducer array (17) and its corresponding focal point (20) is between 40 mm and 80 mm.

10. System (16) according to any one of claims 5-9, wherein the holder (9) comprises an arc-shaped structure (22) that comprises:
- one or more fixing elements (23), each adapted to fix the position and orientation of a FUS transducer array (17) within the arc-shaped structure (22); and
- an arc-shaped rail (24) arranged along the arc-shaped structure (22) adapted to hold the fixing elements (23) within said arc-shaped structure (22);
and wherein the fixing elements (23) are adapted to be released and tightened, such that both the displacement of the FUS transducer arrays (17) along the arc-shaped rail (24) and the relative orientation of the FUS transducer arrays (17) can be modified from at least a first position to a second position.

11. System (16) according to the previous claim, wherein the holder (9) further comprises pivoting means (25) adapted to rotate the arc-shaped structure (22) in the roll, yaw, and pitch degrees of freedom.

12. System (16) according to any one of claims 5-11, wherein the processing means of the control unit (18) comprise hardware and/or software means adapted to:
- process one or more MRI and/or CT images of the subject and segment therein the spinal cord by means of a segmentation algorithm;
- select, according to the previous spinal cord segmentation one or more stimulation points (21) located within the spinal cord of the subject;
- segment the laminae (19) of the subject comprised by the MRI and/or CT images that correspond to the vertebrae surrounding the selected stimulation points (21) by means of a segmentation algorithm and calculate the normal vector to the surface of said laminae (19);
- provide, according to the previous normal vectors, an optimal position of the FUS transducer array/s (17) with respect to the stretcher (1) for each of the selected stimulation points (21) so that the delivery of the FUS waves is maximized;
- calculate, within the MRI and/or CT images, acoustic units, bone absorption, tissue density, speed of the acoustic waves throughout different structures comprised by said images and/or standing waves through the spinal canal; and/or
- determine, one or more spinal cord stimulation parameters including piezoelectric element phases for electronically steering the beam focus and/or aberration control signals;
and wherein the control means are adapted to receive the one or more spinal cord stimulation parameters and to provide instructions to the FUS transducer array/s (17) such that said FUS transducer array/s (17) deliver ultrasound waves as a function of said one or more spinal cord stimulation parameters.

13. Method for optimizing the configuration of a system (16) for non-invasive neurostimulation of the spinal cord of a subject according to any one of claims 5-12, wherein the subject is lying supine on the adjustable stretcher (1),
and **characterized in that** said method comprises performing the following steps:
a) adjusting the position and orientation of the supporting modules (2) of the stretcher (1) according to the subject's body and such that the spine of the subject is arranged within the opening (8) of the stretcher (1);
b) receiving one or more MRI and or CT images of the spinal cord of a subject;
c) processing the images obtained in step b) by using the processing means and:
- identifying a set of anatomical landmarks within the spinal cord of the subject;
- identifying one or more stimulation points (21) within the spinal cord of the subject;
- identifying the laminae (19) of the subject comprised by the MRI and/or CT images that correspond to the vertebrae surrounding the selected stimulation points (21);
d) placing and orienting, according to the anatomical landmarks obtained in step c), the FUS transducer array/s (17) such that the curvature radius direction of each FUS transducer array (17) is perpendicular to a lamina (19) corresponding to the vertebra surrounding an identified stimulation point (21) and such that the focal points (20) of each FUS transducer array (17) substantially coincides with said identified stimulation point (21); and
f) recording the position and orientation of the of the supporting modules (2), the set of anatomical landmarks, the one or more stimulation points (21) and the position and orientation of the FUS transducer array/s (17) for future configurations of the system (16).

14. Method according to the preceding claim further comprising, after step c) and before step f), performing the following steps by using the processing means:
- calculating, within the MRI and/or CT images: acoustic units, bone absorption, tissue density, speed of the acoustic waves throughout different structures comprised by said images and/or standing waves through the spinal canal; and
- determining, from the previous calculation, one or more spinal cord stimulation parameters including piezoelectric element phases for electronically steering the beam focus and/or aberration control signals.

15. Method according to the preceding claim, further comprising, after step c) and before step f), performing the following steps by using the processing means:
- simulating, based on the previous determination, on the measurements of the subject received in step a), on the anatomical landmarks, stimulation points (21) and laminae (19) identified in step c), and on the position and orientation of the FUS transducer array/s (17) in step d) the stimulation effect of the delivery of FUS waves;
- fine-tuning, based on the previous simulation, the values of the one or more spinal cord stimulation parameters; and/or
- sending the fined-tuned values of the one or more spinal cord stimulation parameters to the control means and recording the fined-tuned values of the one or more spinal cord stimulation parameters for future configurations of the system (16).
